(19) Europäisches Patentamt
European Patent Office
Office européen des brevets

(11) Veröffentlichungsnummer: **0 652 205 A2**

# (12) EUROPÄISCHE PATENTANMELDUNG

(21) Anmeldenummer: **94117181.1**

(22) Anmeldetag: **31.10.94**

(51) Int. Cl.⁶: **C07C 59/60**, C07C 59/90, C12P 7/42

(30) Priorität: **04.11.93 DE 4337630**
**26.01.94 DE 4402167**

(43) Veröffentlichungstag der Anmeldung:
**10.05.95 Patentblatt 95/19**

(84) Benannte Vertragsstaaten:
**AT BE CH DE DK ES FR GB GR IE IT LI LU NL PT SE**

(71) Anmelder: **HOECHST AKTIENGESELLSCHAFT**
**Brüningstrasse 50**
**D-65929 Frankfurt am Main (DE)**

(72) Erfinder: **Aretz, Werner, Dr.**
**Am Wäldchen 13**
**D-61462 Königstein (DE)**
Erfinder: **Hedtmann, Udo, Dr.**
**H.-Bleicher-Strasse 35**
**D-60433 Frankfurt (DE)**

## (54) Moenomycin-Abbauprodukfe mit hydroxylierter oder oxidierter Lipidseitenkette und Moenomycin-Analoga, ein Verfahren zur Herstellung und die Verwendung dieser Verbindungen.

(57) Die Erfindung betrifft MA der Formel I

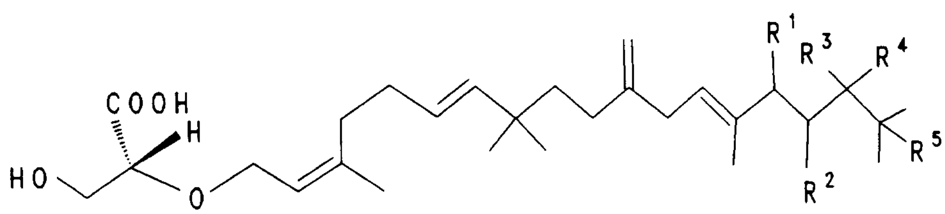

I

in der
$R^1$, $R^2$, $R^4$ und $R^5$ unabhängig voneinander eine Hydroxylgruppe oder Wasserstoff bedeuten und
$R^3$ Wasserstoff bedeutet, wobei die Bindung zwischen dem den Substituenten $R^4$ tragenden C-Atom und dem den Substituenten $R^5$ tragenden C-Atom eine Einfachbindung ist,
oder
$R^1$ und $R^2$ unabhängig voneinander eine Hydroxylgruppe oder Wasserstoff bedeuten und
$R^3$ Wasserstoff bedeutet, wobei die Bindung zwischen dem den Substituenten $R^4$ tragenden C-Atom und dem den Substituenten $R^5$ tragenden C-Atom ist, eine Doppelbindung ist,
oder
$R^1$, $R^2$ und $R^5$ unabhängig voneinander eine Hydroxylgruppe oder Wasserstoff bedeuten und
$R^3$ und $R^4$ zusammen eine Oxogruppe bedeuten.
MA wird durch Mikroorganismen der Gattung Cryptococcus in der Lipidseitenkette hydroxyliert oder oxidiert und ist in dieser Form eine Ausgangsverbindung zur Herstellung von Moenomycin-Analoga, die als Arzneimittel einsetzbar sind.
Die Moenomycin-Analoga haben folgende chemische Struktur:

EP 0 652 205 A2

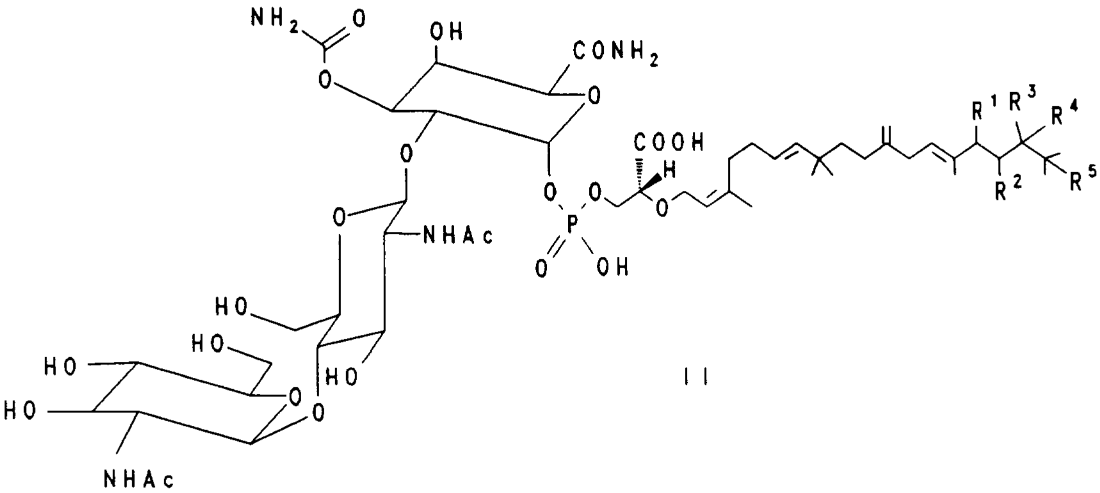

II

in der $R^1$ bis $R^5$ die oben genannten Bedeutungen haben.

Die Erfindung betrifft ein Moenomycin Abbauprodukt (MA), welches durch Mikroorganismen der Gattung Cryptococcus in der Lipidseitenkette hydroxyliert oder oxidiert wird, ein Verfahren zur Herstellung von hydroxyliertem oder oxidiertem MA und von Moenomycin-Analoga auf Basis des oxidierten oder hydroxylierten MAs sowie diese Moenomycin-Analoga selbst, die Verwendung dieser Moenomycin-Analoga als Arzneimittel sowie den Mikroorganismus Cryptococcus albidus DSM 8624.

Moenomycine sind Phosphoglycolipid-Antibiotika, welche die Biosynthese des Peptidglycans der bakteriellen Zellwand durch Inhibition des Enzyms Transglycosylase spezifisch hemmen. Die Moenomycine sind gegen grampositive Keime hochwirksam und erfassen auch MRSA-Keime (Methicillinresistente Staphylococcus aureus-Keime). Moenomycine werden in der Veterinärmedizin, nicht jedoch in der Humantherapie verwendet, da sie nur sehr langsam vom Organismus aus dem Blut eliminiert werden können und damit die Gefahr der Akkumulation von chemischen Verbindungen in Geweben und der Schädigung des Organismus besteht. Für diesen Effekt wird die Lipidseitenkette verantwortlich gemacht.

In der deutschen Patentanmeldung 43 26 777.4 wird ein Verfahren zur Synthese von Transglycosylase-Inhibitoren vorgeschlagen, bei dem durch Verwendung geeigneter Zuckerbausteine und dem gesättigten Glycerinsäureether-Lipid der Moenomycine (gesättigtes MA) antibiotisch wirksame Verbindungen des Moenomycin-Typs hergestellt werden.

Weiterhin ist in der europäischen Patentanmeldung 0 355 679 ein Verfahren zum Abbau der Moenomycine mit den Enzymen Moenomycinase und MBase beschrieben, welches die Abbauprodukte MA, MB und MC liefert. MA, dessen Herstellung in EP 0 503 419 beschrieben ist, besteht aus dem Glycerinsäureether-Lipid der Moenomycine (ungesättigter, verzweigter C-25-Alkohol Moenocinol und Glycerinsäure).

Bislang sind - mit Ausnahme der Hydrierung - Derivatisierungen der Lipidseitenkette weder bei den Moenomycinen noch bei den Abbauprodukten MA oder MB durchgeführt worden.

Aufgabe dieser Erfindung ist die Synthese von Transglycosylase-Inhibitoren für die Humanmedizin mit verbesserten pharmakologischen Eigenschaften.

Die Aufgabe wird erfindungsgemäß gelöst durch die Synthese von Moenomycin-Analoga, indem man Crytococcus sp. fermentiert und der Mikroorganismus das der Fermentationslösung zugesetzte MA in der Lipidseitenkette hydroxyliert oder oxidiert und man nach der Isolierung des derivatisierten MAs aus dem Kulturmedium dieses an Zuckerketten anknüpft.

Die Erfindung betrifft:

1. Eine Verbindung der Formel I

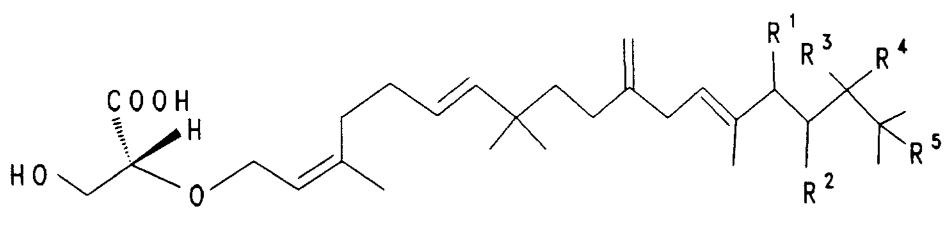

I

in der

$R^1$, $R^2$, $R^4$ und $R^5$ unabhängig voneinander eine Hydroxylgruppe oder Wasserstoff bedeuten und

$R^3$ Wasserstoff bedeutet, wobei die Bindung zwischen dem den Substituenten $R^4$ tragenden C-Atom und dem den Substituenten $R^5$ tragenden C-Atom eine Einfachbindung ist,

oder

$R^1$ und $R^2$ unabhängig voneinander eine Hydroxylgruppe oder Wasserstoff bedeuten und

$R^3$ Wasserstoff bedeutet, wobei die Bindung zwischen dem den Substituenten $R^4$ tragenden C-Atom und dem den Substituenten $R^5$ tragenden C-Atom ist, eine Doppelbindung ist,

oder

$R^1$, $R^2$ und $R^5$ unabhängig voneinander eine Hydroxylgruppe oder Wasserstoff bedeuten und

$R^3$ und $R^4$ zusammen eine Oxogruppe bedeuten.

2. Eine Verbindung der Formel II

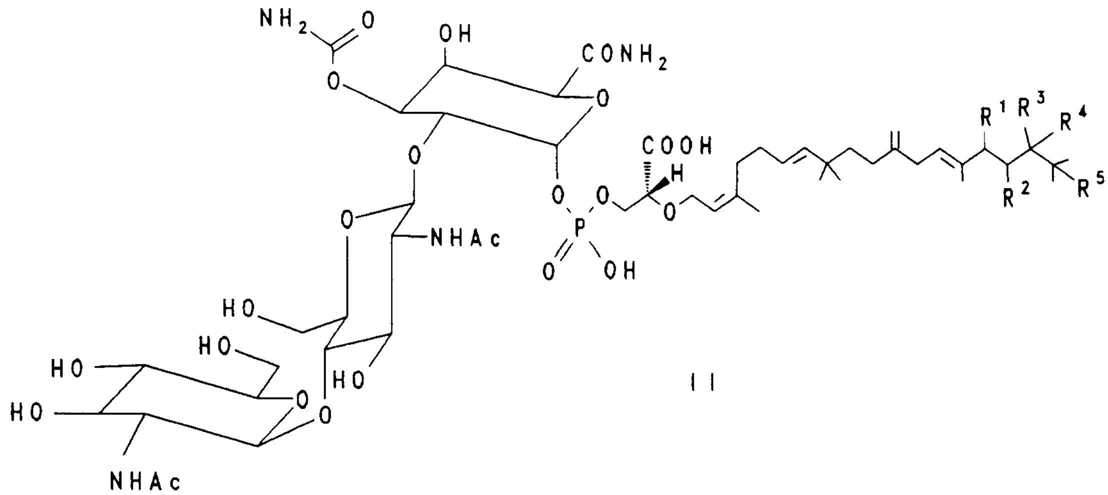

II

in der $R^1$ bis $R^5$ die oben genannten Bedeutungen haben.

3. Arzneimittel, enthaltend eine Verbindung der Formel II und gegebenenfalls pharmazeutische Träger.

4. Ein Verfahren zur Herstellung einer Verbindung der Formel I, das dadurch gekennzeichnet ist, daß man
   - Cryptococcus sp. in einem Kulturmedium unter Zusatz von MA fermentiert,
   - die Verbindung der Formel I aus dem Medium isoliert und gegebenenfalls
   - die Verbindung der Formel I durch ein chromatographisches Verfahren aufreinigt.

5. Ein Verfahren zur Herstellung einer Verbindung der Formel II, dadurch gekennzeichnet, daß man
   - einen n-Alkylester einer Verbindung der Formel I synthetisiert,
   - diesen über eine Phosphatgruppe mit einem Trisaccharid koppelt, welches geschützt ist mit gleichen oder verschiedenen, aus dem Stand der Technik bekannten Schutzgruppen,
   - nachfolgend die Schutzgruppen abspaltet und
   - eine Verbindung der Formel II aufreinigt.

6. Eine Verwendung einer Verbindung der Formel I zur Herstellung von Moenomycin-Analoga.

7. Eine Verwendung einer Verbindung der Formel II als pharmakologisch wirksame Substanz.

8. Cryptococcus albidus DSM 8624.

Im folgenden wird die Erfindung detailliert beschrieben, insbesondere in ihren bevorzugten Ausführungsformen. Ferner wird die Erfindung durch den Inhalt der Ansprüche bestimmt.

Als MA-Analoga bzw. Moenomycin-Analoga werden Verbindungen bezeichnet, die eine im Vergleich zum MA bzw. Moenomycin eine hydroxylierte oder oxidierte Lipidseitenkette besitzen.

Als Schutzgruppe wird eine chemische Gruppe definiert, die funktionelle Gruppen für Reaktionen blockiert und nach Durchführung der Reaktion wieder abgespalten werden kann.

"vvm" ist definiert als 1 Liter Luft pro 1 Liter Nährlösung und Minute.

Für die Synthese von hydroxyliertem oder oxidiertem MA dient MA als Ausgangsverbindung, welches während der Fermentation von Cryptococcus sp. zugesetzt wird. MA ist beispielsweise durch ein in EP 0 503 419 beschriebenes Verfahren erhältlich.

Der Mikroorganismus Cryptococcus albidus wurde am 19.10.1993 bei der Deutschen Sammlung von Mikroorganismen und Zellkulturen , Braunschweig, Mascheroder Weg 1B, Deutschland, unter der Nummer DSM 8624, gemäß den Vorschriften des Budapester Vertrages, hinterlegt.

Der Mikroorganismus Cryptococcus laurenzii DSM 2762 wurde bereits am 20.09.1983 bei der vorgenannten Stelle, gemäß den Vorschriften des Budapester Vertrages, hinterlegt.

Ausgangsmaterial für diese Stämme war eine Erdprobe, die über mehrere Passagen in einem Medium mit D-Glutaminsäure als einziger Stickstoffquelle bei 28 °C für jeweils 2 bis 3 Tage inkubiert wird. Diese Flüssigkulturen werden auf Medien ausplattiert, die D-$\alpha$-Aminoadipinsäure-Ethylamid als einzige N-Quelle enthielten. Nach weiteren Überimpfungen werden die Stämme DSM 2762 und DSM 8624 als Reinkultur isoliert.

Bei Cryptococcus sp. DSM 2762 und DSM 8624 handelt es sich um eine einzellige Hefe, die weder Mycel noch Pseudomycelien bildet. Die Vermehrung vollzieht sich durch vielseitige Sprossung; ein Vorhandensein von Asko- bzw. Ballistosporen kann nicht nachgewiesen werden. Die konvexen, weißlichen Kolonien sind rauh und haben einen glatten Rand. Eine Pigmentbildung in Form von Carotinoiden findet

nicht statt. Der Nachweis von Hefestärke gelingt mit Jod-Jodkalium, und zwar sowohl in den Kolonien als auch in Flüssigkulturen. Physiologische Untersuchungen zeigen, daß Glucose, Saccarose, Maltose, Raffinose, Galaktose, Lactose, Stärke, Rhamnose, Melibiose, Dextrin und Inosit als Kohlenstoffquelle assimiliert werden; eine Vergärung der Zucker findet nicht statt. Eine Verwertung von Ammoniumsulfat, α-Aminoadipinsäure, Glutaminsäure, Alanin, Leucin, Serin, Tryptophan, Tyrosin und Phenylalanin als Stickstoffquelle ist nicht nachweisbar. Ein Wachstum mit Natriumnitrat wird nicht beobachtet.

Ein Mikroorganismus der Gattung Cryptococcus, vorzugsweise Cryptococcus albidus oder Cryptococcus laurenzii, ganz besonders bevorzugt Cryptococcus albidus DSM 8624 oder Cryptococcus laurenzii DSM 2762 wird nach dem Fachmann bekannten Verfahren in einem Fermenter angezogen. Die unterschiedlichen Spezies können gemeinsam oder getrennt fermentiert werden.

Hierzu wird zunächst eine Vorkultur angelegt. Die Medium- und Wachstumsbedingungen sind beispielsweise:

| Vor- und Hauptkulturmedium | |
|---|---|
| Glucose | 2 % |
| Sojamehl | 0,5 % |
| Hefeextrakt | 0,5 % |
| NaCl | 0,5 % |
| $K_2HPO_4$ | 0,5 % |
| pH 7,0 | |

Als Vorkultur dient ein 2 l-Erlenmeyerkolben mit 500 ml Nährlösung, welcher mit einer Abschwemmung vom Schrägröhrchen von Cryptococcus sp. beimpft wird. Nach einer Inkubationszeit von 72 Stunden bei 28°C und einer Schüttelfrequenz von 250 Upm, wird aus dieser Vorkultur die Hauptkultur mit einem Inokulum von 10 % angeimpft. Die Hauptkultur erfolgt in einem 12 l-Fermenter mit 9 l Nährlösung bei 28°C, einer Belüftungsrate von 0,1 vvm und einer Rührergeschwindigkeit von 300 Upm.

MA wird dem Fermenter als Lösung zugesetzt, und der Abbau von MA sowie die Bildung einer Verbindung der Formel I werden dünnschichtchromatographisch nach dem Fachmann bekannten Verfahren verfolgt.

Die Zugabe des Substrats kann zu einem beliebigen Zeitpunkt während des Wachstums oder der stationären Phase von Crytococcus sp. geschehen. Der Stamm kann im wesenlichen auf allen für das Wachstum geeigneten Nährlösung aerob, bevorzugt unter Schütteln, bei 25°C bis 32°C kultiviert werden. Solche Nährmedien können vom Fachmann schnell ohne erfinderisches Zutun gefunden werden.

Vorteilhaft kann man so vorgehen, daß das Substrat (MA) der Mikroorganismenkultur innerhalb des 4-tägigen Wachstums zu der Nährlösung, insbesondere nach 8 bis 48 Stunden und besonders bevorzugt nach 16 bis 30 Stunden, zugesetzt wird. Die zugegebene Menge des Substrats kann in weiten Bereichen schwanken, bevorzugt sind es jedoch 0,1 bis 10 g/l Nährlösung, insbesondere 0,5 g/l Nährlösung. Das Substrat ist vorzugsweise in Methanol vorzulösen. Die Nährlösung enthält vorzugsweise Glucose, Sojamehl und Hefeextrakt als Kohlenstoff- bzw. Stickstoffquelle. Es wird über einen Zeitraum von 70 bis 355 Stunden in dem obengenannten Temperaturbereich inkubiert, wobei der Fortgang der Reduktion mittels DC verfolgt werden kann.

Alternativ zu der oben beschriebenen Methode können zur Hydroxylierung oder Oxidierung auch in physiologischen Puffern gewaschene und aktive Zellen eingesetzt werden. Als Puffer zum Waschen und Aufbewahren der Zellen kann beispielsweise 0,1 bis 1 M Kaliumphosphatpuffer (pH 7.0) verwendet werden.

Nach komplettem Umsatz von MA werden die Zellen abgetrennt (z. B. durch Zentrifugation) und eine Verbindung der Formel I durch Extraktion isoliert.

Im einzelnen werden folgende Verbindungen bevorzugt isoliert: Eine Verbindung der Formel III

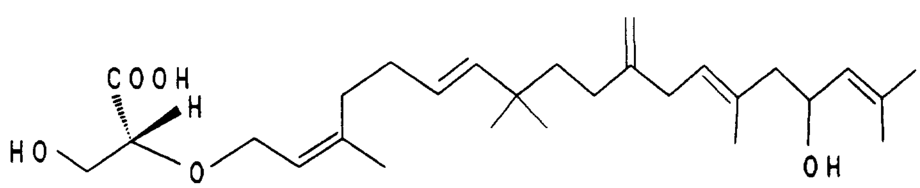

III

($R^1$ = H, $R^2$ = OH, $R^3$ = H, $R^4$ und $R^5$ bilden eine gemeinsame Doppelbindung).

Eine Verbindung der Formel IV

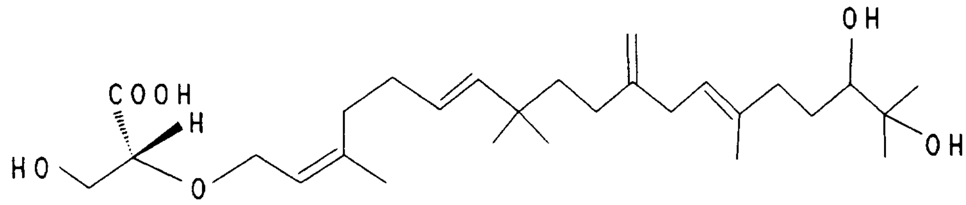

IV

($R^1$ = H, $R^2$ = H, $R^3$ = H, $R^4$ = OH und $R^5$ = OH).

Eine Verbindung der Formel V

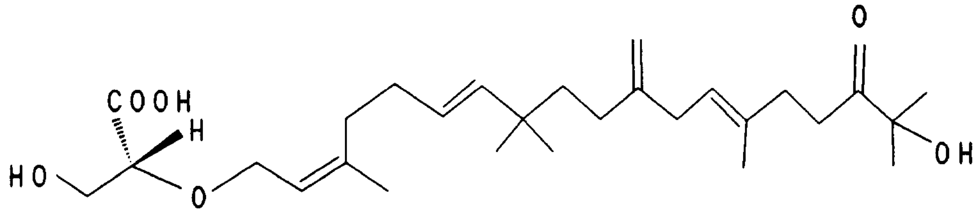

V

($R^1$ = H, $R^2$ = H, $R^3$ und $R^4$ eine Ketogruppe bedeuten und $R^5$ = OH).

Nach der Isolierung durch die Extraktion erhält man ein Gemisch aus Verbindungen der Formel I, vorzugsweise aus Verbindungen der Formeln III, IV und V. Das so erhaltene Rohgemisch wird durch chromatographische Verfahren in seine Einzelkomponenten aufgetrennt. Es wird beispielsweise eine chromatographische Auftrennung mit Kieselgel oder RP-18 (Fa. Merck, Deutschland) durchgeführt. Als Fließmittel dient Chloroform/$CH_3OH$/$H_2O$ im Verhältnis 80:10:1.

Beispielsweise kann die Auftrennung der Verbindungen der Formel I, vorzugsweise die Abtrennung der Verbindung der Formel IV an Kieselgel mit einem Chloroform/Methanol/Essigsäure-Gemisch (Verhältnis 80:10:0,1) erfolgen. Man erhält dann die Verbindung der Formel IV in aufgereinigter Form. Die Trennung und Aufreinigung der Verbindungen der Formeln III und V erfolgt mit Reversed Phase (RP-18-Säule) mit Methanol/Wasser (Verhältnis 5:1), welchem 0,1 % TFA (Trifluroessigsäure) zugesetzt ist.

Eine aufgereinigte Verbindung der Formel I wird dann zur Herstellung von Moenomycin-Analoga (Verbindungen der Formel II) eingesetzt.

Für die Herstellung eines Moenomycin-Analogons aus einer Verbindung der Formel I wird zunächst aus den Verbindungen der Formel I der entsprechende n-Alkylester, vorzugsweise $C_1$-$C_6$-Alkylester und besonders bevorzugt $C_1$-$C_4$ Alkylester, nach dem Fachmann bekannten Verfahren hergestellt, z.B. indem eine Lösung einer Verbindung der Formel I in Methanol mit einem Kationenaustauscher in der $H^+$-Form (z. B. Dowex 50 WX2) versetzt wird und mehrere Stunden (4 Stunden) bei Raumtemperatur (0 bis 50 °C) gerührt wird. Anschließend wird vom Ionenaustauscher abfiltriert, der Rückstand eingeengt und man erhält den entsprechenden Methylester. Gegebenenfalls werden die sekundären OH-Gruppen nach dem Stand der Technik geschützt.

Ein Alkylester einer Verbindung der Formel I wird mit einer nach dem Stand der Technik geschützten Zuckerkette verknüpft. Vorzugsweise sind diese Zuckerketten Di-, Tri- sowie Tetrasaccharide und besonders bevorzugt bestehend aus N-Acetylglucosamin, Chinovosamin, Galacturonsäureamid, Glucuronsäureamid, wobei diese Zucker die Saccaride mit unterschiedlicher Zusammensetzung und Reihenfolge bilden. Die Verknüpfung eines Alkylesters einer Verbindung der Formel I mit einer geschützten Zuckerkette erfolgt über eine Phosphatgruppe, indem vorzugsweise das Phosphitverfahren benutzt wird, bei dem aktivierter Phosphor der Oxidationsstufe III eingesetzt wird (Welzel et al., Tetrahedron 49, 10587 (1993)).

Anschließend werden alle Schutzgruppen abgespalten (je nach verwendeten Schutzgruppen 1 oder 2 Schritte) und man erhält als Endprodukt eine Verbindung der Formel II.

Die zu verwendenden Schutzgruppen sowie die Verfahren zur Einführung oder Abspaltung von Schutzgruppen sind dem Fachmann aus der Literatur bekannt. (Protective Groups in Organic Synthesis, Second Edition, Theodora W. Greene & Peter G.M. Wuts, A Wiley-Interscience Publikum, John Wiley & Sons, Inc., New York, Chichester, Brisbane, Toronto, Singapore.

Bei der Isolierung der Verbindung der Formel II geht man wie folgt vor: Die Isolierung einer Verbindung der Formel II aus dem Reaktionsgemisch erfolgt bevorzugt durch chromatographische Verfahren, besonders bevorzugt an Kieselgel oder RP-18 (Reversed Phase) mit geeigneten Lösungsmittelgemischen, vorzugsweise Isopropanol/Ammoniak im Verhältnis 4:1 oder Methanol/Acetonitril/Wasser im Verhältnis 8:4:1.

Eine Verbindung der Formel II und/oder deren physiologisch verträglichen Salze eignen sich aufgrund ihrer wertvollen pharmakologischen Eigenschaften sehr gut zur Anwendung als Heilmittel. Die Arzneimittel eignen sich vorzugsweise zur Prophylaxe und/oder Therapie von bakteriellen Erkrankungen.

Die erfindungsgemäßen Arzneimittel werden im allgemeinen oral oder parenteral verabreicht, aber auch eine rektale Anwendung ist prinzipiell möglich. Geeignete feste oder flüssige galenische Zubereitungsformen sind beispielsweise Granulate, Pulver, Tabletten, Dragees, (Mikro-)Kapseln, Zäpfchen, Sirupe, Emulsionen, Suspensionen, Aerosole, Tropfen oder injizierbare Lösungen in Ampullenform sowie Präparate mit protrahierter Wirkstoff-Freigabe, bei deren Herstellung üblicherweise Trägerstoffe und Zusätze und/oder Hilfsmittel wie Spreng-, Binde-, Überzugs-, Quellungs-, Gleit- oder Schmiermittel, Geschmacksstoffe, Süßungsmittel oder Lösungsvermittler Verwendung finden. Als häufig verwendete Träger- oder Hilfsstoffe seien z. B. Magnesiumcarbonat, Titandioxid, Laktose, Mannit und andere Zucker, Talkum, Milcheiweiß, Gelatine, Stärke, Vitamine, Cellulose und ihre Derivate, tierische und pflanzliche Öle, Polyethylenglykole und Lösungsmittel, wie etwa steriles Wasser, Alkohole, Glycerin und mehrwertige Alkohole genannt.
Vorzugsweise werden die pharmazeutischen Präparate in Dosierungseinheiten hergestellt und verabreicht, wobei jede Einheit als aktiven Bestandteil eine bestimmte Dosis einer Verbindung der Formel II und/oder deren physiologisch verträglichen Salze enthält. Bei festen Dosierungseinheiten wie Tabletten, Kapseln und Suppositorien, kann diese Dosis bis zu etwa 500 mg, vorzugsweise aber etwa 10 bis 100 mg, betragen.

Für die Behandlung eines erwachsenen Patienten sind - je nach Wirksamkeit einer Verbindung gemäß Formel II und/oder deren physiologisch verträglichen Salze am Menschen - Tagesdosen von etwa 20 bis 500 mg Wirkstoff, vorzugsweise etwa 50 bis 300 mg, bei oraler Verabreichung und von etwa 5 bis 300 mg, bevorzugt etwa 10 bis 100 mg, bei intravenöser Applikation indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrere kleinerer Dosierungseinheiten als auch durch Mehrfachgabe unterteilter Dosen in bestimmten Intervallen erfolgen.
Die erfindungsgemäßen Arzneimittel werden dadurch hergestellt, daß man eine Verbindung der Formel II und/oder deren physiologisch verträglichen Salze mit üblichen Träger- sowie gegebenenfalls Zusatz- und/oder Hilfsstoffen in die bzw. eine geeignete Darreichungsform bringt. Eine Verbindung der Formel II zeigt hervorragende Wirkung als Antibiotikum.

Beispiel 1

Cryptococcus albidus DSM 8624 wird mit 10 ml NaCl vom Schrägröhrchen abgeschwemmt und 5 ml dieser Lösung werden zum Animpfen einer Vorkultur (500 ml Sabouraud Medium in einem 2 l-Kolben) benutzt. Nach einer Inkubationsdauer von 24 Stunden bei 28 °C und 190 Upm wurde ein 12 l-Fermenter mit 8 l der gleichen Nährlösung und mit dieser Vorkultur beimpft und für weitere 24 Stunden bei 28 °C, 300 Upm und einer Belüftungsrate von 0,1 vvm inkubiert. Mit der Zugabe von MA (4 g gelöst in 100 ml 50 % Ethanol) wird die Biotransformation gestartet, deren Verlauf mittels Dünnschichtchromatographie (HPTLC Fertigplatten, Kieselgel $F_{254}$, Laufmittel: $CHCl_3$:MeOH:Eisessig = 80:10:1) verfolgt wird. Nach 143 Stunden ist die Biotransformation beendet.

Beispiel 2

Von der Lösung aus Beispiel 1 werden die Zellen durch Filtration abgetrennt, der Überstand wird dreimal mit Essigester extrahiert und die vereinigten Essigester-Phasen werden am Rotationsverdampfer bis fas zur Trockenheit eingedampft (Ethylacetat-Extrakt). Die Zellen werden zweimal mit Aceton ausgerührt und die Aceton-Phasen ebenfalls eingedampft (Aceton-Extrakt). Beide Extrakte enthalten Biotranformations-Produkte und können für die weitere Reinigung eingesetzt werden.

Beispiel 3

1,9 g des Acetonextraktes werden an 800 ml Kieselgel mit dem Laufmittel $CHCl_3$:MeOH:Eisessig = 80:10:0,1 chromatographiert. Bei einem Fluß von 20 ml/min werden Fraktionen à 10 ml gesammelt. In den Fraktionen 153 bis 180 werden 55 mg Gemisch aus den Verbindungen der Formeln III und V und in den Fraktionen 257 bis 310 41 mg reine Verbindung der Formel IV erhalten.

Verbindung der Formel V: MS (FAB + LiCl) m/z = 493.3 [(M + 2Li-H)$^+$]

Beispiel 4

Das in Beispiel 3 erhaltene Gemisch der Verbindungen der Formeln III und V wird an 50 ml reversed phase Kieselgel (RP-18, Stahlsäule) mit dem Laufmittel MeOH:0.1 % TFA in Wasser = 5:1 chromatographiert. Bei einem Fluß von 2 ml/min wurden Fraktionen mit jeweils 4 ml gesammelt. In den Fraktionen 101 bis 180 werden 8 mg reine Verbindung der Formel V und in den Fraktionen 196 bis 220 21 mg reine Verbindung der Formel III erhalten.
Verbindung der Formel III: MS (FAB + LiCl): m/z = 475,5 [(M + 2Li-H)$^+$]
Verbindung der Formel V: MS (FAB + LiCl): m/z = 491,5 [(M + 2Li-H)$^+$]

Beispiel 5

$^1$H NMR spektroskopische Daten der Verbindung der Formel III
c = 7,8 mg in $CD_3OH$ T = 300 K

| Signal: | δ: | m: |
|---|---|---|
| | 0.965 | (s) |
| | 0.965 | (s) |
| | 1.371 | (m) |
| | 1.374 | (m) |
| | 1.628 | (dt) |
| | 1.652 | (d) |
| | 1.699 | (d) |
| | 1.748 | (q) |
| | 1.900 | (m) |
| | 1.902 | (m) |
| | 2.085 | (dd) |
| | 2.123 | (m) |
| | 2.139 | (m) |
| | 2.278 | (dd) |
| | 2.681 | (m) |
| | 3.720 | (dd) |
| | 3.773 | (dd) |

| Signal: | δ: | m: |
|---|---|---|
| | 3.951 | (dd) |
| | 4.042 | (dd) |
| | 4.179 | (dd) |
| | 4.439 | (ddd) |
| | 4.652 | (m) |
| | 4.663 | (m) |
| | 5.113 | (dqq) |
| | 5.186 | (ddm) |
| | 5.300 | (dt) |
| | 5.373 | (m) |
| | 5.376 | (dt) |

$^{1}H$ NMR spektroskopische Daten der Verbindung der Formel IV
c = 4,5 mg in $CDCl_3$ T = 300 K

| Signal: | δ: | m: |
|---|---|---|
| | 0.97 | (s) |
| | 0.97 | (s) |
| | 1.18 | (s) |
| | 1.22 | (s) |
| | 1.38 | (m) |
| | 1.46 | (m) |
| | 1.61 | (m) |
| | 1.64 | (s) |
| | 1.77 | (s) |
| | 1.89 | (m) |
| | 2.11 | (m) |
| | 2.12 | (m) |
| | 2.13 | (m) |
| | 2.27 | (m) |
| | 2.73 | d(m) |
| | 3.40 | (dd) |

| Signal: | δ: | m: |
|---|---|---|
| | 3.860 | (m) |
| | 3.889 | (m) |
| | 4.01 | (t) |
| | 4.09 | dd(q) |
| | 4.24 | dd(q) |
| | 4.686 | (m) |
| | 4.699 | (m) |
| | 5.24 | (dt) |
| | 5.25 | (t) |
| | 5.35 | (d) |
| | 5.38 | (t) |

$^1H$ NMR spektroskopische Daten der Verbindung der Formel V
c = 7 mg/ml in $CDCl_3$ T = 300 K

| Signal: | δ: | m: |
|---|---|---|
| | 0.97 | (s) |
| | 0.97 | (s) |
| | 1.36 | (m) |
| | 1.39 | (s) |
| | 1.39 | (s) |
| | 1.63 | (s) |
| | 1.72 | (s) |
| | 1.88 | (m) |
| | 2.05 | (s) |
| | 2.05 | (s) |
| | 2.33 | (t) |
| | 2.67 | (t) |
| | 2.69 | (d) |
| | 3.82 | (dd) |
| | 3.83 | (dd) |
| | 3.91 | (dd) |

| Signal: | δ: | m: |
|---|---|---|
| | 4.07 | (m) |
| | 4.67 | (d) |
| | 4.69 | (d) |
| | 5.20 | (t) |
| | 5.27 | (dt) |
| | 5.35 | (t) |
| | 5.37 | (dt) |

**Patentansprüche**

**1.** Verbindung der Formel I

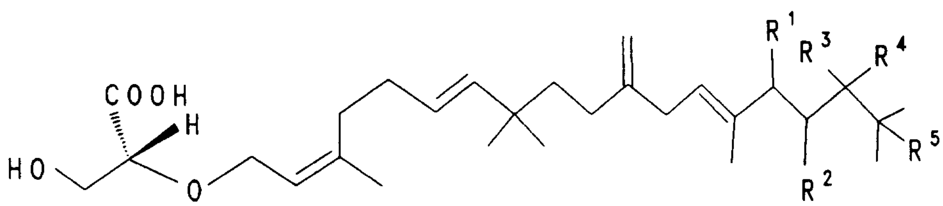

I

in der
$R^1$, $R^2$, $R^4$ und $R^5$ unabhängig voneinander eine Hydroxylgruppe oder Wasserstoff bedeuten und $R^3$ Wasserstoff bedeutet, wobei die Bindung zwischen dem den Substituenten $R^4$ tragenden C-Atom und dem den Substituenten $R^5$ tragenden C-Atom eine Einfachbindung ist,
oder
$R^1$ und $R^2$ unabhängig voneinander eine Hydroxylgruppe oder Wasserstoff bedeuten und $R^3$ Wasserstoff bedeutet, wobei die Bindung zwischen dem den Substituenten $R^4$ tragenden C-Atom und dem den Substituenten $R^5$ tragenden C-Atom ist, eine Doppelbindung ist,
oder $R^1$, $R^2$ und $R^5$ unabhängig voneinander eine Hydroxylgruppe oder Wasserstoff bedeuten und $R^3$ und $R^4$ zusammen eine Oxogruppe bedeuten.

**2.** Verbindung der Formel II

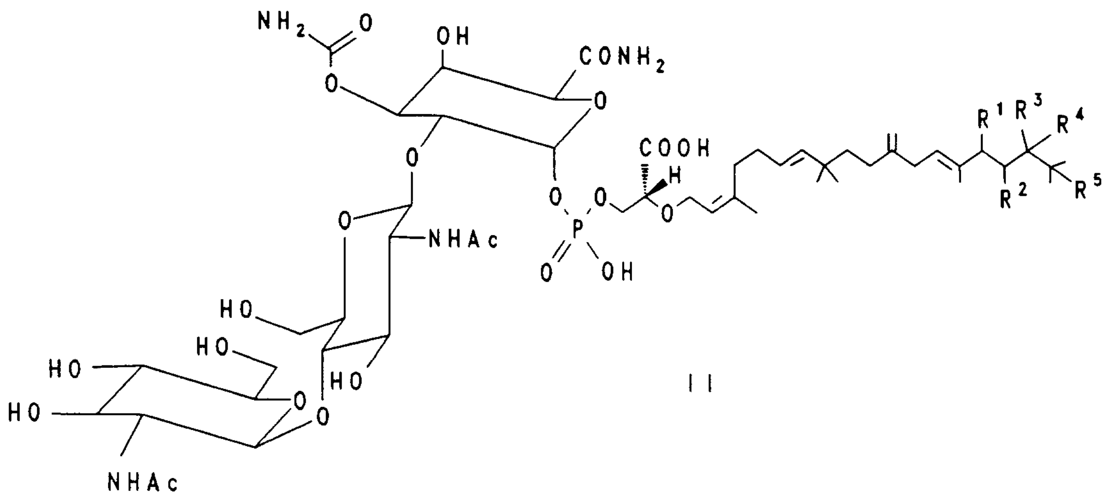

II

in der $R^1$ bis $R^5$ die in Anspruch 1 genannten Bedeutungen haben.

**3.** Arzneimittel, enthaltend eine Verbindung der Formel II und gegebenenfalls pharmazeutische Träger.

**4.** Verfahren zur Herstellung einer Verbindung der Formel I, dadurch gekennzeichnet, daß man
- Cryptococcus sp. in einem Kulturmedium unter Zusatz von MA fermentiert,
- eine Verbindung der Formel I aus dem Medium isoliert und gegebenenfalls
- eine Verbindung der Formel I durch ein chromatographisches Verfahren aufreinigt.

**5.** Verfahren nach Anspruch 4, dadurch gekennzeichnet, daß die Konzentration von MA in der Nährlösung 0,1 g/l - 10 g/l Nährlösung ist.

**6.** Verfahren nach Anspruch 4, dadurch gekennzeichnet, daß Mikroorganismen der Spezies Cryptococcus albidus und/oder Cryptococcus laurentii fermentiert werden.

**7.** Verfahren nach Anspruch 6, dadurch gekennzeichnet, daß Cryptococcus albidus DSM 8624 und/oder Cryptococcus laurentii DMS 2762 fermentiert wird.

**8.** Verfahren zur Herstellung einer Verbindung der Formel II gemäß Anspruch 2, dadurch gekennzeichnet, daß man
- einen n-Alkylester einer Verbindung der Formel I synthetisiert,
- diesen über eine Phosphatgruppe mit einem Trisaccharid koppelt, welches geschützt ist mit gleichen oder verschiedenen, aus dem Stand der Technik bekannten Schutzgruppen,
- nachfolgend die Schutzgruppen abspaltet und
- eine Verbindung der Formel II aufreinigt.

**9.** Verwendung einer Verbindung der Formel I gemäß Anspruch 1 zur Herstellung von Moenomycin-Analoga.

**10.** Verwendung einer Verbindung der Formel II gemäß Anspruch 2 als pharmakologisch wirksame Substanz.

**11.** Verwendung gemäß Anspruch 10, dadurch gekennzeichnet, daß eine Verbindung der Formel II als Antibiotikum verwendet wird.

**12.** Cryptococcus albidus DSM 8624.